# EUROPEAN PATENT SPECIFICATION

(11) **EP 1 786 763 B1**
(45) Date of publication and mention of the grant of the patent: **16.09.2009**
(21) Application number: 05732459.2
(22) Date of filing: 15.04.2005
(51) Int. Cl.: C07C 277/08, C07C 279/12, C11C 3/10

(54) **N, N', N"-TRIS-(3-DIMETHYLAMINOPROPYL)-GUANIDINE, THE PROCEDURE OF PREPARATION FROM CARBODIIMIDE AND APPLICATION IN REACTIONS OF TRANSESTERIFICATION OF OIL**
N, N', N"-TRIS-(3-DIMETHYLAMINOPROPYL)-GUANIDIN, HERSTELLUNGSVERFAHREN AUS CARBODIIMID UND ANWENDUNG IN REAKTIONEN ZUR UMESTERUNG VON ÖL
N, N', N''-TRIS-(3-DIMETHYLAMINOPROPYL)-GUANIDINE, PROCEDE DE PREPARATION ET APPLICATION DANS LA TRANSESTERIFICATION D'HUILES VEGETALES

(30) Priority: 15.04.2004 HR 20040341
(43) Date of publication of application: 23.05.2007
(73) Proprietor: RUDJER BOSKOVIC INSTITUTE, HR-10002 Zagreb (HR)
(72) Inventor: MAKSIC, Mirjana, 10000 Zagreb (HR); GLASOVAC, Zoran, 10290 Zapresic (HR)
(74) Representative: von Füner, Nicolai
(86) International application number: PCT/HR2005/000026
(87) International publication number: WO 2005/100306

(56) References cited:
- SCHUCHARDT U ET AL: "ALKYLGUANIDINES AS CATALYSTS FOR THE TRANSESTERIFICATION OF RAPESEED OIL" JOURNAL OF MOLECULAR CATALYSIS, LAUSANNE, CH, vol. 99, 1995, pages 65-70, XP000992192 cited in the application
- KOVACEVIC, BORISLAV ET AL: "The intramolecular hydrogen bond and intrinsic proton affinity of neutral organic molecules: N,N',N''-tris(3-aminopropyl)guanidine and some related systems" JOURNAL OF PHYSICAL ORGANIC CHEMISTRY, 15(11), 765-774 CODEN: JPOCEE; ISSN: 0894-3230, 2002, XP008049476

## Description

The present invention relates to the preparation of N,N',N''-tris-(3-dimethylaminopropyl)-guanidine from N¹,N³-bis-(3-dimethylaminopropyl)-carbodiimide and its use as catalyst for transesterification of vegetable oils.

Transesterification of vegetable oils using light organic alcohols, such as e.g. methanol or ethanol, which provides basis for production of technologically important "Biodiesel^{™}" fuel, is usually carried out in the presence of highly basic anorganic catalysts. The procedures are reviewed in F. Ma, M. A. Hanna, Biores. Technol. 1999, 70, 1-15. The most active among them is sodium methoxide which is, however, not suitable for industrial use as it requires absolute water-free conditions (U. Schuchardt, R. M. Vargas, G. Gelbard, J. Mol. Catal. A: Chemical, 1995, 99, 65-70). Thus most of the currently used commercial procedures employ sodium or potassium hydroxide or carbonate or a mixture of these compounds, as they are very active and relatively cheap. Efficacy of such processes is, however, critically dependent on the presence of free acids in the oil and the water content in the alcohol, which might cause hydrolysis of the esters with subsequent formation of soaps or emulsion from the alcochol/oil mixture (F. Ma, M. A. Hanna, Biores. Technol. 1999, 70, 1-15; U. Schuchardt, R. M. Vargas, G. Gelbard, J. Mol. Catal. A: Chemical, 1995, 99, 65-70; G. Vincente, M. Martinez, J. Aracil, Biores. Technol. 2004, 92, 297-305.).

The possibility of using guanidine derivatives as catalysts in transesterification of various vegetable oils has been discussed in several previously published scientific papers (G. Gelbard, F. Vielfaure-Joly, React. Funct. Polym. 2001, 48, 65-74; G. Gelbard, F. Vielfaure-Joly, Tetrahedron Lett. 1998, 39, 2743-2746). Most of these studies were confined to usage of pentasubstituted guanidine derivatives (G. Gelbard, F. Vielfaure-Joly, React. Funct. Polym. 2001, 48, 65-74; G. Gelbard, F. Vielfaure-Joly, Tetrahedron Lett. 1998, 39, 2743-2746; G. Gelbard, F. Vielfaure-Joly, React. Funct. Polym. 2001, 48, 65-74; U. Schuchardt, R M. Vargas, G. Gelbard, J. Mol. Catal. A: Chemical, 1996, 109, 37-44; G. Gelbard, F. Vielfaure-Joly, C. R. Acad. Sci. Paris, Ser. IIc, Chimie/Chemistry, 2000, 3, 563-567) and polysubstituted biguanides (U. Schuchardt, R. M. Vargas, G. Gelbard, J. Mol. Catal. A: Chemical, 99 (1995) 65-70). More recently, use of some guanidine derivatives bound to the polystyrene support was also described (G. Gelbard, F. Vielfaure-Joly, React. Funct. Polym. 2001, 48, 65-74; U. Schuchardt, R. M. Vargas, G. Gelbard, J. Mol. Catal. A: Chemical, 1996, 109, 37-44; G. Gelbard, F. Vielfaure-Joly, C. R. Acad. Sci. Paris, Ser. IIc, ChimielChemistry, 2000, 3, 563-567). This early work provided a strong evidence that efficacy of catalytic activity of guanidine derivatives increases by increasing basicity, as well as by increasing the number and nature of substituents at the guanidine moiety. Among trisubstituted guanidines only catalytic activity of N,N,N'-tricyclohexyl-guanidine and N¹,N³-dicyclohexyl-N²-n-octylguanidine has been, to the best of our knowledge, tested so far.

Recent theoretical studies have shown that trisubstituted guanidines with the possibility of forming intramolecular hydrogen bond (IMHB) between guanidine subunit and side-chain substitutent, as for example N,N',N"-tris-(3-dimethylaminopropyl)-guanidine **(1),** which is prepared according to this invention, possess higher intrinsic basicity than non-IMHB derivatives, making them interesting targets for investigation of catalytic activity in transesterification reaction. For example, according to the recent *ab initio* calculations, compound 1 was found to possess gas-phase basicity of 275 kcal/mol, while its p*Kₐ* value in acetonitile was predicted to be 29. Both of these values are higher than those of previously studied guanidine catalysts (B. Kova 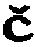 evi 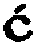 , Z. Glasovac, Z.M. Maksic, , *J. Phys. Org. Chem*. 15, 2002, 765-774). Literature search indicated that neither the preparation of compound **1** nor its use as catalyst for transesterification reactions have been reported in literature so far.

The primary goal of this invention is the preparation of N,N',N"-tris-(3-dimethylaminopropyl)-guanidine **(1),** which was predicted to possess high basicity by computations. The second goal was the usage of this novel trisubstituted guanidine derivative as a catalyst in transesterification reactions of vegetable oils like rapeseed, sunflower and soybean oil.

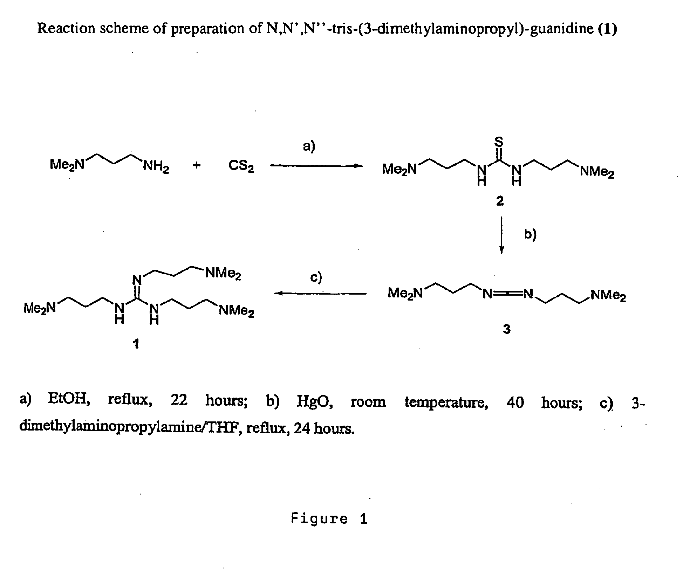

Preparation of N,N',N"-tris-(3-dimethylaminopropyl)-guamdine **(1)** described in this patent application involves three steps, two of which have been previously described in literature: A. F. McKay, D. L. Garmaise, H. A. Baker, L. R. Hawkins, V. Falta, R. Gaudry, G. Y. Paris, J. Med. Chem. 1963, 6, 587-595, desulfiuization was performed according to: J. C. Sheehan, P. A. Cruickshank, G. L. Boshart, J. Org. Chem. 1961, 26, 2525-2528. The first step involves preparation of N¹,N³-bis-(3-dimethylaminopropyl)-thiourea (**2**) from 3-dimethylaminopropylamine and carbon disulfide. Subsequently, N¹,N³-bis-(3-dimethylaminopropyl)-thiourea was transformed into N¹,N³-bis-(3-dimethylaminopropyl)-carbodiimide (3) by reacting with yellow mercury oxide. The third step, **which is a subject matter of the present invention,** comprises addition of 3-dimethylaminopropylamine to N¹, N³-bis-(3-dimethylaminopropyl)-carbodiimide (**3**). **Figure 1****.**

Catalytic activity of the so obtained N,N',N''-tris-(3-dimethylaminopropyl)-guanidine was tested in transesterification reactions of vegetable oils as illustrated by the Examples given below.

A further subject matter of the present invention is the transesterification of rapeseed oil, sunflower oil, soybean oil and other vegetable oils, wherein the N,N',N'''-tris-(3-dimethylaminopropyl)-guanidine, which is prepared according to the present invention, is used as catalyst.

### Detailed description of preparation of N,N',N''-tris-(3-dimethylaminopropyl)-guanidine

### Preparation of starting materials

**N¹,N³-Bis-(3-dimethylaminopropyl)-thiourea (2).** To the solution of CS₂ (6.0 cm³, 7.60 g, 0.100 mol) in 100 cm³ of ethanol cooled to approximately 10 °C, 3-dimethylaminopropylamine (25.2 cm³, 20.50 g, 0.200 mol) was added dropwise. Alter completition of addition, the reaction mixture was refluxed for 22 h. H₂S developed during reaction was absorbed in diluted aqueous NaOH. Solvent and volatile compounds were removed under reduced pressure. Obtained viscous oil (20.5 g, 0.083 mol, Yield = 83 %) was used in the next step without further purification. **¹H NMR (CDCl₃)** δ/ppm: 1.65-1.71 (m) 4H; 2.20 (s) 12H; 2.28-2.36 (m) 4H; 3.4 (broad multiplet) 4H; **¹³C NMR (CDCl₃)** δ/ppm: 25.8 (d); 40.6 (d); 45.0 (t); 57.8 (d); 181.3 (q)

**N¹,N³-Bis(3-dimethylaminopropyl)-carbodiimide (3).** To the solution of thiourea 2 (23.04 g, 0.094 mol) in 150 cm³ CH₂Cl₂ previously dried over CaH₂ yellow HgO (80.0 g, 0.369 mol) was added in one portion. Heterogeneous mixture was stirred for 22 hours at room temperature, then the second portion of yellow HgO (20.0 g, 0.092 mol) was added and the stirring continued for additional 18 hours. Then the reaction mixture was cooled to room temperature, filtered and the solvent evaporated at reduced pressure. Oily product was distilled at 0.1 Pa (10⁻⁴ mmHg) keeping temperature of the oil bath below 100 °C. GLC analysis of colorless distillate showed the presence of the carbodiimide **3** as the single product (7.33 g, 0.035 mol, Yield = 37 %, b.p. = 48-50°C /3x 10⁻⁴ mmHg). **GC-MS** (M⁺ m/z = 212); **IR**(KBr) ( *ṽ*/cm⁻¹ = 2130, N=C=N); **¹H NMR (CDCl₃)** δ/ppm: 1.68-1.77 (m), 4H; 2.22 (s) 12H; 2.31-2.36 (t) 4H; 3.23-3.31 (t) 4H; **¹³C NMR (CDCl₃)** δ/ppm: 29.3 (d); 44.8 (d); 45,5 (t); 56.9 (d); 140.1 (q)

### Process of the invention

**N,N',N''-Tris(3-dimethylaminopropyl)-guanidine (1):** Solution of carbodiimide 3 (7.33 g, 0.035 mol), and 3-dimethylaminopropyl-1-amine (8.70 g, 0.070 mol) in 40.0 cm³ of dry THF was refluxed over 24 hours. After cooling the reaction mixture to room temperature, solvent and excess of amine were evaporated under reduced pressure. Obtained crude colorless oily product (10.75 g, 0.034 mol, Yield = 99 %) was of sufficient purity for use in transesterification reactions. Analytically pure sample was obtained by a distillation of the crude product at 0.01 Pa (10⁻⁵ mmHg, b.p. = 132-136 °C / 2 x 10⁻⁴ mmHg). **IR**(KBr) (*ṽ*/cm⁻¹ = 1584, 1628; N=C(NH)₂); **HR-MS** (MH⁺) exp.: m/z = 315.322185 (teor. m/z = 315.32307); **¹H NMR (CDCl₃)** δ/ppm: 1.59-1.68 (m) 6H; 2.14 (s) 18H; 2.25-2.29 (t, *J* = 6 Hz) 6H; 3.09-3.13 (t, *J* = 6 Hz) 6H; **¹³C NMR (CDCl₃)** δ/ppm: 27.5 (d); 39.3 (d); 45.2 (t); 57.3 (d); 159.2 (q)

**Transesterification of sunflower oil:** 4.00 g of sunflower oil, 3.00 cm³ of methanol and 5 molar percent of guanidine 1 was heated at 70 °C. Samples of the reaction mixture were taken after 1, 2, 3 and 4 hours, washed with brine (three times) and dried over MgSO₄, product composition was analyzed by ¹H-NMR spectroscopy (CDCl₃, using TMS as standard). Percentage of the transesterified product was determined from the intensity ratio of methyl ester signals (δ = 3.8 ppm) and signals corresponding to the α- methylene protons (8 = 2.3 ppm). Analysis of the recorded ¹H-NMR spectra showed that all of the starting compound underwent transesterification within lh of the reaction time.

It would be obvious to one skilled in the art that guanidine **1,** which is prepared according to this invention, can be used in transesterification reaction of other vegetable oils.

## Claims

1. Preparation of N,N',N''-tris(3-dimethylaminopropyl)-guanidine comprising addition of 3-dimethylaminopropyl-1-amine to N¹,N³-bis-(3-dimethylaminopropyl)-carbodiimide,
**characterized in that** each of the nitrogen atoms in the guanidine moiety is substituted with 3-dimethylaminopropyl group.

2. Transesterification of rapeseed oil, sunflower oil, soybean oil and other vegetable oils in methanol, **wherein** the N,N',N"-tris-(3-dimethylaminopropyl)-guanidine, which is prepared according to claim 1, is used as catalyst.

## Patentansprüche

1. Herstellung von N,N',N"-tris-(3-dimethylaminopropyl)-guanidin durch Zugabe von 3-Dimethylaminopropyl-1-amin zu N¹,N³-bis-(3-dimethylaminopropyl)-carbodiimind,
**dadurch gekennzeichnet, daß** jedes der Stickstoffatome im Guanidinfragment mit der 3-Dimethylaminopropylgruppe substituiert ist.

2. Umesterung von Raps-, Sonnenblumen- und Sojabohnenöl und anderer Pflanzenöle in Methanol, **wobei** das gemäß Anspruch 1 hergestellte N,N',N"-tris-(3-dimethylaminopropyl)-guanidin als Katalysator verwendet wird.

## Revendications

1. Préparation de la N,N',N"-tris(3-diméthylaminopropyl)-guanidine comprenant l'addition de la 3-diméthylaminopropyl-1-amine à du N¹,N³-bis-(3-diméthylaminopropyl)-carbodiimide, **caractérisée en ce que** chacun des atomes d'azote dans la fraction de guanidine est substitué avec un groupe 3-diméthylaminopropyle.

2. Transestérification d'huile de colza, d'huile de tournesol, d'huile de soja et d'autres huiles végétales dans du méthanol, dans laquelle la N,N',N"-tris(3-diméthylaminopropyl)-guanidine, que l'on prépare conformément à la revendication 1, est utilisée comme catalyseur.
